(19) Europäisches Patentamt
European Patent Office
Office européen des brevets

(11) Publication number: **0 160 563**
**A2**

(12) **EUROPEAN PATENT APPLICATION**

(21) Application number: 85303030.2

(22) Date of filing: 29.04.85

(51) Int. Cl.⁴: C 07 D 501/46
//C07C131/00

(30) Priority: 30.04.84 GB 8410991

(43) Date of publication of application: 06.11.85 Bulletin 85/45

(84) Designated Contracting States: AT BE CH DE FR GB IT LI NL SE

(71) Applicant: GLAXO GROUP LIMITED
Clarges House 6-12 Clarges Street
London W1Y 8DH(GB)

(72) Inventor: Looker, Brian Edgar
28 Middleton Avenue
Greenford Middlesex(GB)

(74) Representative: Pett, Christopher Phineas et al,
Frank B.Dehn & Co. Imperial House 15-19 Kingsway
London WC2B 6UZ(GB)

(54) **Process for preparing cephalosporin compounds.**

(57) There is described a process for the preparation of compounds of general formula (I)

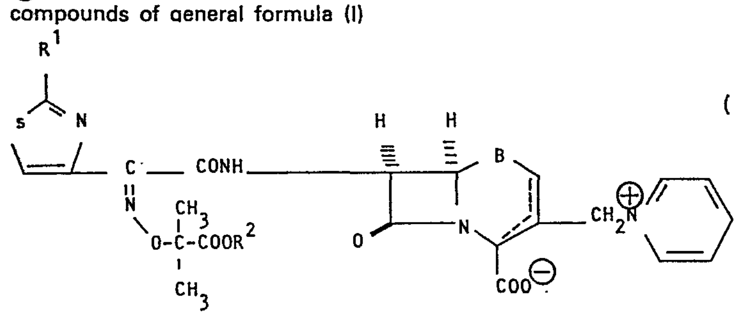

(I)

(wherein $R^1$ is an amino or protected amino group, $R^2$ is hydrogen or a carboxyl blocking group, B is >S or >S → O and the dotted line bridging the 2-, 3- and 4- positions indicates that the compound is a ceph-2-em or ceph-3-em compound) or a corresponding compound having a group of formula $COOR^3$ at the 4- position (wherein $R^3$ is a carboxyl blocking group) and having an associated anion $A^{\ominus}$, and salts thereof, which comprises reacting a compound of general formula (II)

./...

EP 0 160 563 A2

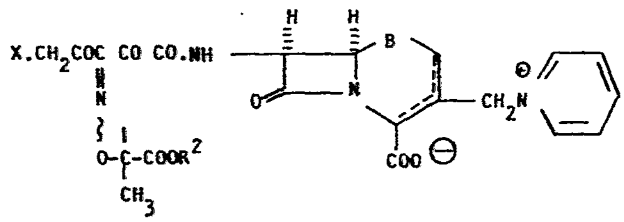

(II)

(wherein $R^2$, B and the dotted line are as defined above, X is a leaving group and the wavy line indicates that the compound may be in the *syn* and /or *anti* configuration) or a salt thereof or a corresponding compound having a group of formula $-COOR^3$ at the 4-position (wherein $R^3$ is as defined above) and having an associated anion $A^{\ominus}$, with a compound of formula (III)

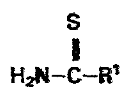

(III)

(wherein $R^1$ is as defined above). The process is of value as a means of preparing the antibiotic compound ceftazidime. Intermediates of value in the process and methods of preparing them are also disclosed.

"Process for preparing cephalosporin compounds"

This invention relates to improvements in or relating to cephalosporins. More particularly it relates to processes for the preparation of the cephalosporin antibiotic ceftazidime.

Ceftazidime ((6R,7R)-7-[(Z)-2-(2-aminothiazol-4-yl)-2-(2-carboxyprop-2-oxyimino)acetamido]-3-(1-pyridiniummethyl)ceph-3-em-4-carboxylate) is a valuable antibiotic having broad spectrum activity, in particular, unusually high activity against gram-negative organisms, including Pseudomonas organisms, as described in UK Patent Specification No. 2025398.

Semi-synthetic cephalosporins may conventionally be prepared by the acylation of an appropriate 7-aminocephalosporin with a compound serving to introduce a pre-formed 7-substituent, or by modification at the 3-position, for example the reaction of a 3-acyloxymethyl or 3-halomethyl cephalosporin with a nucleophile, to introduce the desired 3-substituent. These general processes are described in UK Patent Specification No. 2025398 for the preparation of ceftazidime.

We have now devised a process for the preparation of ceftazidime and salts and protected derivatives thereof in which the 2-aminothiazol-4-yl ring in the 7-substituent of the cephalosporin compound is formed as the last major chemical stage in the synthesis.

According to one aspect of the invention, therefore, we provide a process for the preparation of compounds of general formula (I)

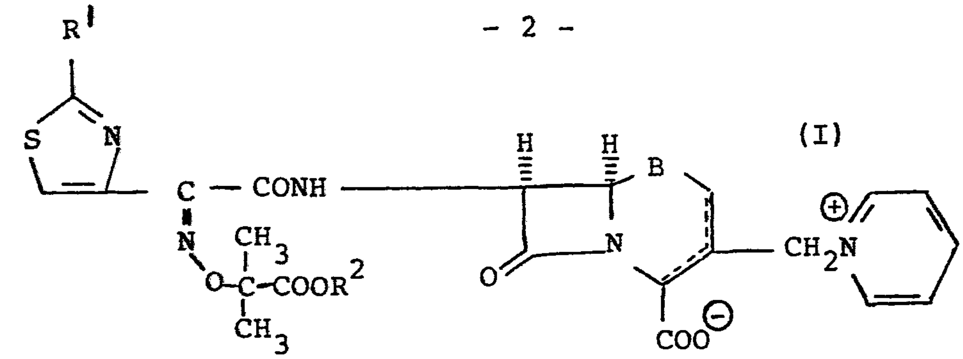

(I)

(wherein $R^1$ is an amino or protected amino group, $R^2$ is hydrogen or a carboxyl blocking group, B is >S or >S→O and the dotted line bridging the 2-, 3- and 4- positions indicates that the compound is a ceph-2-em or ceph-3-em compound) or a corresponding compound having a group of formula $COOR^3$ at the 4- position (wherein $R^3$ is a carboxyl blocking group) and having an associated anion $A^{\ominus}$, and salts thereof, which comprises reacting a compound of general formula (II)

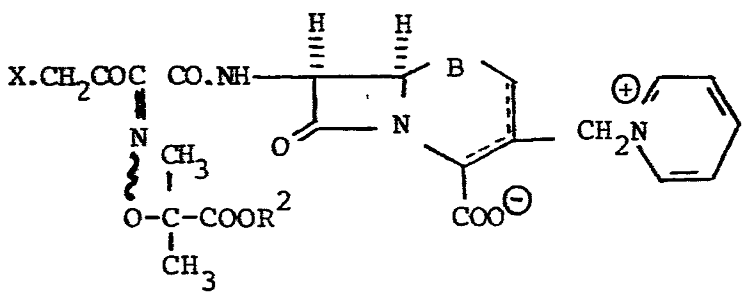

(II)

(wherein $R^2$, B and the dotted line are as defined above, X is a leaving group and the wavy line indicates that the compound may be in the <u>syn</u> and/or <u>anti</u> configuration) or a salt thereof or a corresponding compound having a group of formula $-COOR^3$ at the 4-position (wherein $R^3$ is as defined above) and having an associated anion $A^{\ominus}$, with a compound of formula (III)

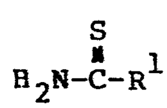

(III)

(wherein $R^1$ is as defined above). The compound of formula (III) is preferably thiourea, i.e. $R^1$ represents an amino group.

Examples of the leaving group X include halogen atoms e.g. chlorine, bromine or iodine, and hydrocarbyl (e.g. aryl or aliphatic) sulphonyloxy groups such as p-toluenesulphonyloxy, methanesulphonyloxy or trifluoromethanesulphonyloxy.

The anion $A^{\ominus}$ which may be associated with a compound of formula (I) or (II) may be derived from an organic or inorganic acid, such as an alkyl-, aryl- or aralkyl- carboxylic or -sulphonic acid or a mineral acid. Examples of such acids include formic, trifluoroacetic, methanesulphonic, p-toluenesulphonic, hydrochloric, hydrobromic, phosphoric and sulphuric acids.

The reaction may be effected in either aqueous or non-aqueous reaction media. Suitable reaction media include alcohols such as methanol or ethanol; ethers, e.g. cyclic ethers such as tetrahydrofuran or dioxan, or acyclic ethers such as diethylether or diglyme; amides, such as N,N-dimethylformamide, N,N-dimethylacetamide or hexamethylphosphoramide; esters, such as ethyl acetate; nitriles such as acetonitrile; nitroalkanes such as nitromethane; sulphoxides, such as dimethylsulphoxide; sulphones such as sulpholane; and hydrocarbons, especially halogenated hydrocarbons such as methylene chloride; which solvents may optionally contain water, as well as mixtures of two or more such solvents. Reaction will conveniently be carried out at a temperature from -50 to +100°C preferably at from 0 to 50°C.

The reaction medium may if desired incorporate an acid binding agent e.g. a tertiary amine such as triethylamine, diisopropylmethylamine or dimethylaniline; an amide such as acetamide; an inorganic base such as calcium carbonate or sodium bicarbonate;

or an oxirane such as ethylene oxide or propylene oxide. Alternatively molecular sieves may be used. The acid binding agent is preferably a tertiary amine.

The reaction with the compound of formula (III) may be followed where necessary and/or desired by conversion of the compound of formula (I) initially obtained into a different compound of formula (I), for example by means of one or more of the following reactions:-

(i) reduction of a compound wherein B is $>S\rightarrow O$ to a compound wherein B is S;

(ii) conversion of a $\Delta^2$-isomer into a $\Delta^3$-isomer;

(iii) removal of any carboxyl-blocking or amino protecting groups; and

(iv) formation of a non-toxic salt or a non-toxic metabolically labile ester.

These reactions may be effected by conventional methods and in any convenient order.

Thus, if desired, a $\Delta^2$-cephalosporin obtained in accordance with the process of the invention may be converted into the corresponding $\Delta^3$- derivative by, for example, treatment of the $\Delta^2$- derivative with a base, such as pyridine or triethylamine.

A ceph-2-em reaction product may also be oxidised to yield the corresponding ceph-3-em 1-oxide, for example by reaction with a peracid, e.g. peracetic or m-chloroperbenzoic acid; the resulting sulphoxide may subsequently be reduced as described hereinafter to yield the corresponding ceph-3-em sulphide.

Where a compound of formula (I) is obtained in which B is $>S\rightarrow O$ this may, if desired, be converted

into the corresponding sulphide by, for example, reduction of the corresponding acyloxysulphonium or alkoxysulphonium salt prepared in situ by reaction with e.g. acetyl chloride in the case of an acetoxysulphonium salt, reduction being effected by, for example, sodium dithionite or by iodide ion as in a solution of potassium iodide in a solvent e.g. acetic acid, acetone, tetrahydrofuran, dioxan, dimethylformamide or dimethylacetamide. The reaction may be effected at a temperature of from -20° to +50°C.

Carboxyl blocking groups which may be present in the compounds of general formula (I) or any of the intermediates described herein may be any of the conventional carboxyl protecting groups known in the art, a list of representative protected carboxyl groups being included in British Patent No. 1399086. The carboxyl blocking group may be for example the residue of an ester-forming aliphatic or araliphatic alcohol or an ester-forming phenol, silanol or stannanol preferably containing 1-20 carbon atoms. Thus, carboxyl blocking groups which may conveniently be employed include aryl lower alkyl groups, such as p-methoxybenzyl, p-nitrobenzyl and diphenylmethyl; lower alkyl groups such as t-butyl; lower haloalkyl groups such as 2,2,2-trichloroethyl; and tri(lower alkyl) silyl groups such as trimethylsilyl.

An amino protecting group present in the compounds of formula (I) or intermediates therefor is conveniently a group which may be readily removed at an appropriate stage in the reaction sequence, for example an aralkyl group, such as triphenylmethyl, or an acyl group, such as formyl, t-butoxycarbonyl, 2,2,2-trichloroethoxycarbonyl or isoamyloxycarbonyl.

Any carboxyl blocking or amino protecting group present in the compound of formula (I) may

be removed, if desired, by any appropriate methods known in the art. Suitable methods for removing carboxyl protecting groups include acid or base hydrolysis and reduction. Thus, for example diphenylmethyl and t-butyl carboxyl blocking groups may conveniently be removed by acid hydrolysis. A p-nitrobenzyl group may conveniently be cleaved by reduction, effected for example by zinc and acetic acid, or sodium dithionite or iodide ion in water, conveniently in the presence of a water-miscible solvent such as acetone, tetrahydrofuran, dioxan, dimethylformamide or dimethylacetamide.

Methods of removing amino protecting groups are also well known in the art. A trityl group may for example be removed using an optionally halogenated carboxylic acid e.g. acetic acid, formic acid, chloroacetic acid or trifluoroacetic acid or using a mineral acid, e.g. hydrochloric acid or mixtures of such acids, preferably in the presence of a protic solvent such as water. Acid-labile acyl amino-protecting groups e.g. formyl, t-butoxycarbonyl or isoamyloxycarbonyl may also be removed, where appropriate, under the conditions described above for a trityl group. Other acyl amino-protecting groups e.g. 2,2,2-trichloroethoxycarbonyl may be removed, for example, by reduction.

It will be appreciated that the use of amino protecting and carboxyl blocking groups is well known in the art. Relevant examples of such use are given in e.g. Theodora W. Greene "Protective Groups in Organic Synthesis" (Wiley-Interscience, New York 1981), and J.F.W. McOmie, "Protective Groups in Organic Chemistry" (Plenum Press, London 1973).

Salt derivatives of the compounds of formula (I) include inorganic base salts such as alkali metal salts (e.g. sodium and potassium salts) and

alkaline earth metal salts (e.g. calcium and magnesium salts); ammonium salts; amino acid salts (e.g. lysine and arginine salts); organic base salts (e.g. procaine, phenylethylbenzylamine, dibenzylethylenediamine, ethanolamine, diethanolamine and N-methylglucosamine salts), and acid addition salts, e.g. formed with a mineral acid such as hydrochloric, hydrobromic, sulphuric, nitric or phosphoric acid, or with an organic acid such as formic, acetic, trifluoroacetic, methanesulphonic or p-toluenesulphonic acid. The salts may also be in the form of resinates formed with, for example, a polystyrene resin or cross-linked polystyrene divinylbenzene copolymer resin containing amino or quaternary amino groups or sulphonic acid groups, or with a resin containing carboxyl groups, e.g. a polyacrylic acid resin.

It will be appreciated that when the compound of formula (I) prepared according to the invention is ceftazidime itself, salt and ester derivatives should be non-toxic and physiologically acceptable, such as are described in UK Patent Specification No. 2025398.

Base salts of formula (I) may be formed by reacting an acid of formula (I) with an appropriate base. Thus, for example, sodium or potassium salts may be prepared using the respective 2-ethylhexanoate or hydrogen carbonate salt. Acid addition salts may be prepared by reacting a compound of formula (I) with the appropriate acid.

The reaction product may be separated from the reaction mixture, which may contain, for example, unchanged cephalosporin starting material and other substances, by a variety of processes including solvent extraction, recrystallisation, ionophoresis, column chromatography and use of ion-exchangers (for example by chromatography on ion-exchange resins) or macroreticular resins.

Where a compound of formula (I) is obtained as a mixture of isomers, the syn isomer may be obtained by, for example, conventional methods such as crystallisation or chromatography.

A preferred embodiment of the process of the invention relates to a process for the preparation of ceftazidime itself or an ester thereof, i.e. the compound of formula (I) in which $R^1$ represents an amino group, $R^2$ represents a hydrogen atom or an esterifying group, the dotted line represents a ceph-3-em compound and B is >S, followed if desired by removal of the esterifying group, optionally in situ.

Compounds of formula (II) as defined above and their salts are novel compounds and according to a further aspect of the invention we provide compounds of formula (II) as defined above and salts thereof, and processes for their preparation.

The compounds of formula (II) may be prepared, for example, by acylating a compound of formula (IV)

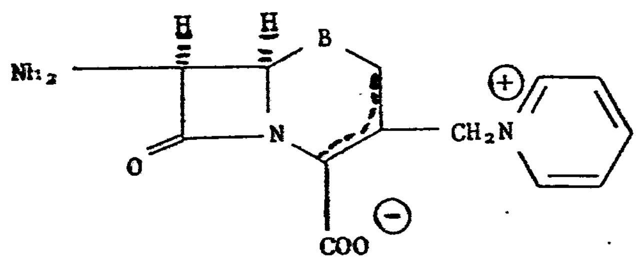

(IV)

(wherein B and the dotted line are as defined above) or a salt e.g. an acid addition salt or N-silyl derivative thereof or a corresponding compound having a group of formula $COOR^3$ at the 4-position and an associated anion $A^{\ominus}$, (wherein $A^{\ominus}$ and $R^3$ are as defined above) with an acid of formula (V)

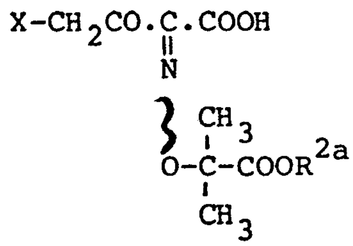

(V)

(wherein X and the wavy line are as defined above, and $R^{2a}$ represents a carboxyl blocking group) or an acylating agent corresponding thereto, followed if desired by removal of the carboxyl protecting group $R^{2a}$. The acylation reaction may be effected by conventional methods, for example as described in British Patent No. 2025398.

Thus acylating agents corresponding to the acid of formula (V) which may be employed include acid halides, e.g. acid chlorides or bromides, activated esters, and symmetrical or mixed anhydrides.

Acylation may also be effected using an acid of formula (V), in which case a condensing agent such as N,N'-dicyclohexylcarbodiimide, carbonyldiimidazole or N-ethyl-5-phenylisoxazolium perchlorate, is desirably present in the reaction mixture.

Alternatively, an activated form of an acid of formula (V) for use in the acylation reaction may be formed _in situ_ by reacting an acid of formula (V) with a solution or suspension of a halogenating agent preformed by adding a carbonyl halide, e.g. oxalyl chloride or phosgene or a phosphoryl halide, e.g. phosphorus oxychloride, to a solvent such as a halogenated hydrocarbon, e.g. methylene chloride, containing a lower acyl tertiary amide such as N,N-dimethylformamide.

The acylation reaction may conveniently be effected in a suitable solvent or mixture of solvents, such as an alcohol e.g. aqueous ethanol or aqueous industrial methylated spirit at temperatures from -50 to +50°C, if desired in the presence of an acid binding agent.

Compounds of formula (V) as defined above, and their salts and reactive derivatives are novel compounds and according to a further aspect of the invention we provide compounds of formula (V) as defined above and salts and reactive derivatives thereof, and processes for their preparation.

The compounds of formula (V) may be prepared for example by reacting a compound of formula (VI)

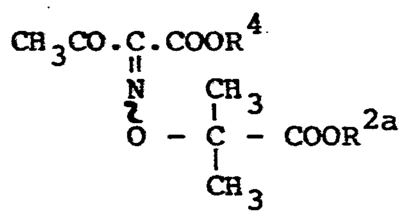

(VI)

(wherein $R^4$ represents hydrogen or a carboxyl blocking group and $R^{2a}$ and the wavy line are as defined above) with a compound serving to introduce the group X as defined above. To prepare compounds of formula (V) wherein X represents bromine or chlorine the compound of formula (VI) may be reacted with the appropriate halogenating agent such as bromine or sulphuryl chloride in a suitable solvent, for example a halogenated hydrocarbon such as methylene chloride or chloroform. Compounds of formula (V) wherein X represents iodine may be prepared by reacting a corresponding bromo- or chloro- compound with iodide ion, using for example a solution of sodium or potassium iodide in a solvent such as acetone. When $R^4$ represents a carboxyl blocking group the preparation of a compound of formula (V) is preferably effected under acidic conditions such that the carboxyl blocking group $R^4$ is removed during the course of the reaction. It will be appreciated that the carboxyl blocking group $R^{2a}$ should be a group which is less readily cleaved by acid hydrolysis than is the group $R^4$,

such that the resulting compound of formula (V) possesses a blocked carboxyl group in the oxime moiety. A particularly useful blocking group $R^{2a}$ is the p- nitrobenzyl group. $R^4$ preferably represents t-butyl.

Compounds of formula (VI) as defined above and their salts are also novel compounds and according to a yet further aspect of the invention we provide compounds of formula (VI) as defined above and salts thereof, and processes for their preparation.

Compounds of formula (VI) may be prepared for example by etherifying a compound of formula (VII)

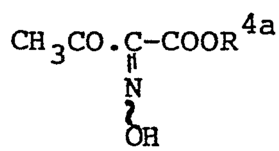

(VII)

(wherein the wavy line is as defined above and $R^{4a}$ is a carboxyl blocking group) with a compound of formula (VIII)

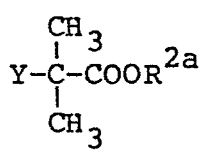

(VIII)

(wherein $R^{2a}$ is as defined above and Y is a halogen atom, such as chloro, bromo or iodo; sulphate; or sulphonate such as tosylate), followed if desired by selective removal of the blocking group $R^{4a}$. The etherification reaction is generally carried out in the presence of a base e.g. potassium carbonate or sodium hydride and in an organic solvent, e.g. acetone, tetrahydrofuran, dimethylformamide or dimethylsulphoxide.

The invention will now be more particularly described in the following Examples. In the Examples,

all temperatures are in °C. Sorbsil U30 is silica gel manufactured by Joseph Crosfield and Son of Warrington, Cheshire, England. Amberlite XAD-2 is a non-functional macroreticular resin manufactured by Rohm and Haas of Philadelphia USA.

Example 1

t-Butyl 2-[2-(4-nitrobenzyloxycarbonyl)-2-methylpropionyloxyimino]-3-oxobutyrate

t-Butyl 2-hydroxyimino-3-oxobutyrate (18.7 g) was stirred with 4-nitrobenzyl 2-bromoisobutyrate (34.5 g) in acetone (200 ml) containing potassium carbonate (45 g) under nitrogen for two hours at 21°. The mixture was partitioned between methylene chloride and water and the aqueous layer was extracted with more methylene chloride. The combined organic layers were washed with water, dried with magnesium sulphate and evaporated to an oil (44 g). A portion of this oil was chromatographed on Sorbsil U30 (200 g) in ethyl acetate (10 to 25%) in petroleum ether (bp 40 to 60°) to give the title compound (10.7 g), τ ($CDCl_3$) 1.81 and 2.48 (2d, J9Hz; aromatic protons), 4.68 (s; $OCH_2$), 7.78 (s; $CH_3CO$), 8.37 (s; $(CH_3)_2$), and 8.46 (s; t-butyl).

Example 2

4-Bromo-2-[2-(4-nitrobenzyloxycarbonyl)-2-methylpropionyloxyimino]-3-oxobutyric acid

The product of Example 1 (10.2 g) was dissolved in methylene chloride (250 ml) and bromine (1.42 ml) was added with stirring. Hydrogen bromide was passed into the solution for three minutes. The solution was stirred at 21° for four hours. The solution was decanted from a gummy deposit and the solvent was evaporated to give the title compound (12.74 g), τ ($CDCl_3$) 0.83 (s; COOH), 1.76 and 2.48 (2d, J 9Hz; aromatic protons), 4.65 (s; $OCH_2$), 5.83 (s; $BrCH_2$) and 8.30 (s; $(CH_3)_2$).

Example 3

(6R,7R)-7-{4-Bromo-2-[2-(4-nitrobenzyloxycarbonyl)-2-methylpropionyloxyimino]-3-oxobutyramido}-3-(1-pyridiniummethyl)ceph-3-em-4-carboxylate

Oxalyl chloride (0.37 ml) was added to a

solution of N,N-dimethylformamide (0.38 ml) in methylene chloride (10 ml) at -20°. The mixture was stirred with ice-water cooling for five minutes before recooling to -20°. The product of Example 2 (1.73 g) was added in methylene chloride (5 ml) and the resulting solution was stirred with ice-water cooling for 10 minutes before recooling to -20°. This solution was added to a solution of (6R,7R)-7-amino-3-(1-pyridiniummethyl)ceph-3-em-4-carboxylate dihydrochloride dihydrate (1.52 g) in industrial methylated spirits (12 ml), and water (3 ml) containing triethylamine (2.35 ml) at -10°. The solution was allowed to warm to 21° over 15 minutes. It was partitioned between methylene chloride and water and the aqueous layer was extracted with more methylene chloride. The combined organic layers were washed with water containing a little brine. After drying with magnesium sulphate, the solution was evaporated to a foam which was triturated with diethyl ether to give the <u>title compound</u> (1.22 g) λmax (pH 6 buffer) 257.5 nm ($E^{1\%}_{1cm}$ 163), λinfl 274 nm ($E^{1\%}_{1cm}$ 120).

<u>Example 4</u>

<u>[6R,7R]-7-[(Z)-2-(2-Aminothiazol-4-yl)-2-(2-carboxyprop-2-oxyimino)acetamido]-3-(1-pyridiniummethyl)-ceph-3-em-4-carboxylate</u>

The product of Example 3 (352 mg) was dissolved in ethanol (5 ml), water (1 ml) and tetrahydrofuran (5 ml) and N,N-dimethylaniline (0.2 ml) was added. Thiourea (42 mg) was added and the mixture was stirred for five hours. The supernatant solution was decanted from a gum and evaporated to dryness. The residue was triturated with diethyl ether to give a solid (290 mg). A portion of this solid (250 mg) was dissolved in tetrahydrofuran (10 ml), water (6 ml) and saturated sodium bicarbonate solution (4 ml). Sodium dithionite (250 mg) was added and

the solution was stirred at 21° for 20 minutes. The mixture was evaporated and the residue was triturated with acetone to give a solid (700 mg). This was dissolved in water and loaded onto a column of Amberlite XAD-2 resin (100 g). The column was eluted first with water (to remove inorganic impurities) and then with 25% ethanol in water. Evaporation of the latter eluate and trituration with acetone gave the title compound (61 mg) as a solid. The product was shown to contain ceftazidime by comparison of infra-red and nuclear magnetic resonance spectra with spectra of an authentic sample which resembled those obtained in Examples 2 and 4(b) of UK Patent Specification No. 2025398, and by high pressure liquid chromatographic analysis.

CLAIMS:

1. A process for the preparation of compounds of general formula (I)

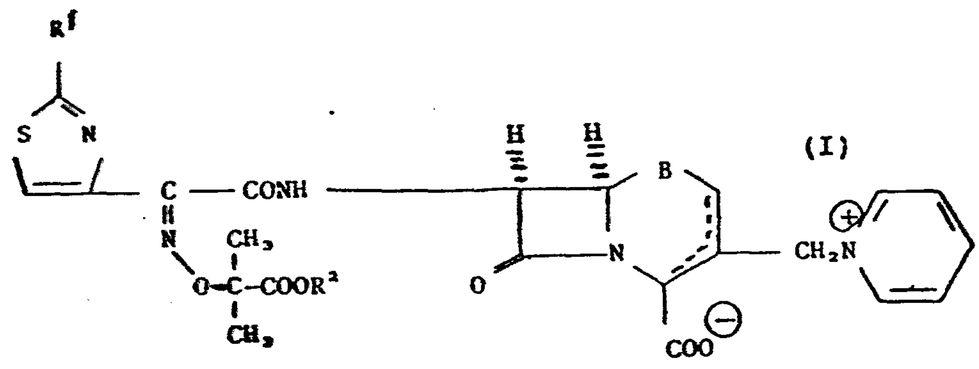

(I)

(wherein $R^1$ is an amino or protected amino group, $R^2$ is hydrogen or a carboxyl blocking group, B is >S or >S →O and the dotted line bridging the 2-, 3- and 4- positions indicates that the compound is a ceph-2-em or ceph-3-em compound) or a corresponding compound having a group of formula $COOR^3$ at the 4- position (wherein $R^3$ is a carboxyl blocking group) and having an associated anion $A^{\ominus}$, and salts thereof, which comprises reacting a compound of general formula (II)

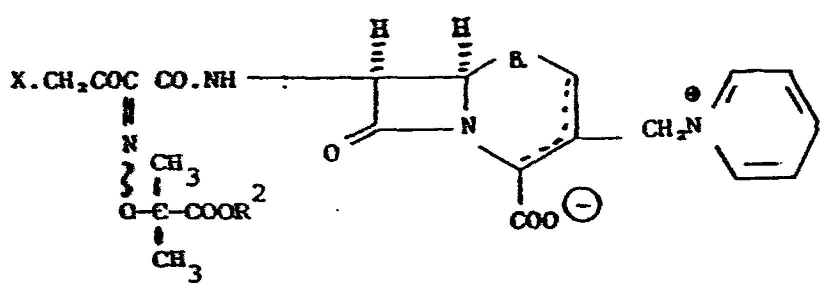

(II)

(wherein $R^2$, B and the dotted line are as defined above, X is a leaving group and the wavy line indicates that the compound may be in the syn and/or anti configuration) or a salt thereof or a corresponding compound having a group of formula $-COOR^3$ at the 4-position (wherein $R^3$ is as defined above) and having an associated anion $A^{\ominus}$, with a compound

of formula (III)

$$H_2N-\overset{\overset{S}{\|}}{C}-R^1 \qquad \text{(III)}$$

(wherein $R^1$ is as defined above).

2. A process as claimed in claim 1 wherein the compound of formula (III) is thiourea.

3. A process as claimed in claim 1 or claim 2 wherein reaction is effected in an alcohol, ether, amide, ester, nitrile, nitroalkane, sulphoxide, sulphone or hydrocarbon solvent, or a mixture of two or more thereof, optionally in the presence of water.

4. A process as claimed in any of claims 1 to 3 wherein reaction is carried out in the presence of an acid binding agent.

5. A process as claimed in any of claims 1 to 4 wherein the reaction of the compound of formula (II) with the compound of formula (III) is followed, where necessary or desired, by conversion of the compound of formula (I) initially formed into a different compound of formula (I) by means of one or more of the following reactions

(i) reduction of a compound wherein B is $>S \rightarrow O$ to a compound wherein B is $>S$;

(ii) conversion of a $\Delta^2$-isomer into a $\Delta^3$-isomer;

(iii) removal of any carboxyl-blocking or amino protecting groups; and

(iv) formation of a non-toxic salt or a non-toxic metabolically labile ester.

6. A process as claimed in any of claims 1 to 5 wherein in the compound of formula (I) obtained,

$R^1$ represents an amino group, $R^2$ represents hydrogen atom or esterifying group, the dotted line represents a ceph-3-em compound and B is >S.

7. A compound of formula (II) as defined in claim 1.

8. A process for the preparation of a compound of formula (II) as defined in claim 1 which comprises acylating a compound of formula (IV)

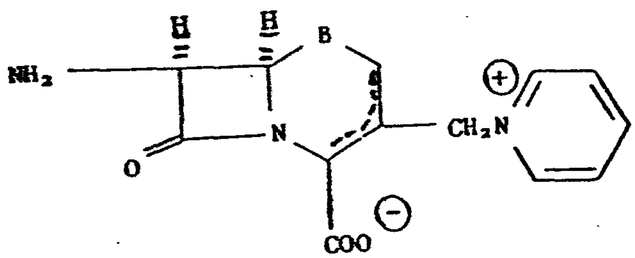

(IV)

(wherein B and the dotted line are as defined above) or a salt e.g. an acid addition salt or N-silyl derivative thereof or a corresponding compound having a group of formula $COOR^3$ at the 4-position and an associated anion $A^{\ominus}$, (wherein $A^{\ominus}$ and $R^3$ are as defined in claim 1) with an acid of formula (V)

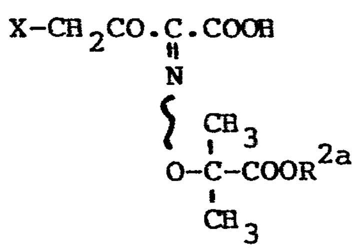

(V)

(wherein X and the wavy line are as defined above, and $R^{2a}$ represents a carboxyl blocking group) or an acylating agent corresponding thereto, followed if desired by removal of the carboxyl protecting group $R^{2a}$.

9. A process as claimed in claim 8 wherein acylation is carried out using an activated form of an acid of formula (V) as defined in claim 8 which is formed <u>in situ</u>.

10. A compound of formula (V) as defined in claim 8 or a salt or reactive derivative thereof.

11. A compound of formula (VI)

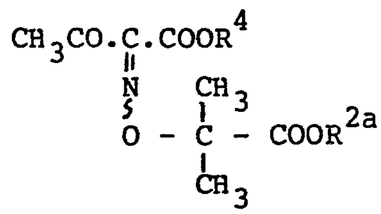

(VI)

wherein $R^4$ represents hydrogen or a carboxyl blocking group, $R^{2a}$ represents a carboxyl blocking group and the wavy line indicates that the compound may be in the <u>syn</u>- and/or <u>anti</u>-configuration, or a salt thereof.